# EUROPEAN PATENT APPLICATION

(11) **EP 3 446 618 A1**
(43) Date of publication of application: **27.02.2019**
(21) Application number: 17785667.1
(22) Date of filing: 03.03.2017
(51) Int. Cl.: A61B 1/00

(54) **ENDOSCOPE SYSTEM, PROCESSOR DEVICE, AND ENDOSCOPE SYSTEM OPERATION METHOD**

(30) Priority: 21.04.2016 JP 2016085336
(71) Applicant: FUJIFILM Corporation, Tokyo 106-8620 (JP)
(72) Inventor: ENDO, Maiko, Ashigarakami-gun Kanagawa 258-8538 (JP)
(74) Representative: Klunker IP Patentanwälte PartG mbB
(86) International application number: PCT/JP2017/008425
(87) International publication number: WO 2017/183324

(57) **Abstract**

Provided are an endoscope system, a processor device, and an endoscope system operation method enabling a focused blood vessel to be easily observed in diagnosis. An endoscope system (10) includes a light source unit (20) that is able to emit a plurality of types of illumination light having different wavelengths, a blood vessel selection part (77) that selects a blood vessel from an image of an observation target imaged by using the illumination light, or from an image generated on the basis of an image of an observation target imaged by using the illumination light, a blood vessel depth estimation part (78) that estimates a depth of the blood vessel selected by the blood vessel selection part (77), and a wavelength changing part (79) that changes a wavelength of the illumination light by using the depth of the blood vessel estimated by the blood vessel depth estimation part.

## Description

### BACKGROUND OF THE INVENTION

### 1. Field of the Invention

The present invention relates to an endoscope system including a light source capable of emitting a plurality of types of illumination light having different wavelengths, a processor device thereof, and an endoscope system operation method.

### 2. Description of the Related Art

In a medical field, generally, diagnosis is performed by using an endoscope system including a light source device, an endoscope, and a processor device. Particularly, in recent years, an endoscope system has also been widespread which has not only an observation mode in which an observation target is observed in a natural shade but also an observation mode in which so-called special light such as blue and green narrowband light beams having a considerably narrow wavelength range are used as illumination light, and thus a blood vessel or the like is easily observed.

In a medical field, an observation target of an endoscope system is present in a living body, and thus is not irradiated with external light. Thus, the observation target is irradiated with illumination light generated by a light source device from a tip end portion of an endoscope, and is imaged by using reflected light thereof or the like. However, since illuminance changes depending on a distance (hereinafter, referred to as an observation distance) or an orientation (angle) between the tip end portion of the endoscope and the observation target, in a case where an amount of illumination light is made constant, a captured image obtained by imaging the observation target by using the endoscope or an observation image which is generated and displayed by using the captured image may not have desired brightness. Thus, in the endoscope system, typically, the brightness of a captured image or the like is monitored, an amount of illumination light is controlled, and thus an observation target can be imaged at substantially constant brightness regardless of an observation distance or the like.

In recent years, an endoscope system has been proposed not only in which an amount of illumination light is simply changed, but also in which a blue component or a green component of the illumination light is changed according to an observation distance so that an image in which a desired structure or the like is easily observed is obtained (JP2015-195844A). There is an endoscope system in which a tone of illumination light is changed depending on an observation distance, an amount of reflected light, or an observation part (JP2014-014716A). There is also an endoscope system in which a narrowband light having a narrow wavelength range is used, and light amount shortage is made up for by increasing a half-value width of blue or green narrowband light during observation in a distant view (JP2012-045266A).

### SUMMARY OF THE INVENTION

In recent diagnosis using an endoscope system, there has been a case where the diagnosis is performed by focusing on a blood vessel within a range of a specific depth direction (hereinafter, referred to as a specific depth) with a surface of the mucous membrane or the like as a reference according to the type of lesion. However, in an endoscope system of the related art, it is hard to observe a focused blood vessel separately from other blood vessels (non-focused blood vessels) depending on a depth of the focused blood vessel in diagnosis.

For example, in an endoscope system of the related art in which a blood vessel or the like is emphasized by using special light as illumination light, a blood vessel to be emphasized is defined by a wavelength of the special light, and the wavelength of the special light is predefined through setting. Thus, in a situation in which a focused blood vessel and other blood vessels are emphasized together due to the wavelength of the special light, it may be hard to focus on the focused blood vessel separately from the other blood vessels. In an endoscope system of the related art in which a tone or the like of illumination light is changed on the basis of an observation distance, a focused blood vessel may be hardly observed since the tone or the like of the illumination light is changed regardless of the focused blood vessel.

An object of the present invention is to provide an endoscope system, a processor device, and an endoscope system operation method enabling a focused blood vessel to be easily observed regardless of a depth of the focused blood vessel.

According to the present invention, there is provided an endoscope system comprising a light source unit that is able to emit a plurality of types of illumination light having different wavelengths; a blood vessel selection part that selects a blood vessel from an image of an observation target imaged by using the illumination light, or from an image generated on the basis of an image of an observation target imaged by using the illumination light; a blood vessel depth estimation part that estimates a depth of the blood vessel selected by the blood vessel selection part; and a wavelength changing part that changes a wavelength of the illumination light by using the depth of the blood vessel estimated by the blood vessel depth estimation part.

The wavelength changing part preferably changes the wavelength of the illumination light to a shorter wavelength as the estimated depth of the blood vessel becomes smaller.

The blood vessel selection part preferably classifies blood vessels which are selectable from an image of the observation target or from an image generated on the basis of an image of the observation target imaged by using the illumination light, on the basis of thicknesses of the blood vessels, and selects a blood vessel having a thickness of which an appearance frequency is highest.

The endoscope system preferably further comprises an observation distance calculation part that calculates an observation distance indicating a distance at which the observation target is imaged from an image of the observation target or from an image generated on the basis of an image of the observation target imaged by using the illumination light, and the blood vessel selection part determines a blood vessel to be selected by using the observation distance.

The blood vessel selection part preferably selects a blood vessel at a shallower position as the observation distance becomes shorter.

The blood vessel selection part preferably selects a thinner blood vessel as the observation distance becomes shorter.

The endoscope system may further comprise an input unit that inputs an instruction for designating a blood vessel to be selected to the blood vessel selection part.

The input unit is preferably a graphical user interface.

The endoscope system preferably has a plurality of observation modes in which the type of illumination light which is used or a combination of illumination light beams differs, and the blood vessel selection part preferably selects a predefined blood vessel in each of the observation modes.

The blood vessel depth estimation part preferably estimates a depth of the blood vessel selected by the blood vessel selection part by using a database.

The blood vessel depth estimation part preferably estimates a depth of the blood vessel selected by the blood vessel selection part by using contrast, brightness, or a color of the blood vessel selected by the blood vessel selection part.

The endoscope system preferably further comprises an image acquisition unit that acquires a plurality of images of the observation target obtained by imaging the observation target at timings at which the plurality of types of the illumination light having different wavelengths are applied by using the plurality of illumination light having different wavelengths and applied at different timings, and the blood vessel selection part preferably selects a blood vessel by using a single or plural images among the plurality of images of the observation target, and the blood vessel depth estimation part estimates a depth of the blood vessel selected by the blood vessel selection part by using a single or plural images among the plurality of images of the observation target.

According to the present invention, there is provided a processor device comprising a blood vessel selection part that selects a blood vessel from an image of an observation target imaged by using illumination light, or from an image generated on the basis of an image of an observation target imaged by using the illumination light; a blood vessel depth estimation part that estimates a depth of the blood vessel selected by the blood vessel selection part; and a wavelength changing part that changes a wavelength of the illumination light by using the depth of the blood vessel estimated by the blood vessel depth estimation part.

According to the present invention, there is provided an endoscope system operation method for an endoscope system including a light source unit that is able to emit a plurality of types of illumination light having different wavelengths, the endoscope system operation method comprising a step of causing a blood vessel selection part to select a blood vessel from an image of an observation target imaged by using the illumination light, or from an image generated on the basis of an image of an observation target imaged by using the illumination light; a step of causing a blood vessel depth estimation part to estimate a depth of the blood vessel selected by the blood vessel selection part; and a step of causing a wavelength changing part to change a wavelength of the illumination light by using the depth of the blood vessel estimated by the blood vessel depth estimation part.

According to the endoscope system, the processor device, and the endoscope system operation method of embodiments of the present invention, since a wavelength of illumination light is changed by using a depth of a blood vessel selected from an image of an observation target, or from an image generated on the basis of an image of the observation target imaged by using illumination light, a focused blood vessel is easily observed.

### BRIEF DESCRIPTION OF THE DRAWINGS

Fig. 1 is an exterior diagram of an endoscope system.
Fig. 2 is a block diagram of the endoscope system.
Fig. 3 is a block diagram of a light source unit.
Fig. 4 is a block diagram of a special processing portion.
Fig. 5 is a graph illustrating a relationship among a wavelength of illumination light, a depth of a blood vessel, and contrast.
Fig. 6 is a graph illustrating a relationship among a wavelength of illumination light, a depth of a blood vessel, and contrast.
Fig. 7 is a flowchart illustrating a special observation mode.
Fig. 8 illustrates a monitor on which a blood vessel emphasis image before a wavelength of illumination light is changed is displayed.
Fig. 9 illustrates the monitor on which a blood vessel emphasis image after a wavelength of illumination light is changed is displayed.
Fig. 10 is a block diagram of a special processing portion in a second embodiment.
Fig. 11 is a graph illustrating an appearance frequency for a thickness of a blood vessel.
Fig. 12 is a block diagram of a special processing portion in a third embodiment.
Fig. 13 is a block diagram of the special processing portion in a case where a depth of a blood vessel is estimated by using a database.
Fig. 14 is a schematic diagram of a capsule endoscope.

### DESCRIPTION OF THE PREFERRED EMBODIMENTS

### [First Embodiment]

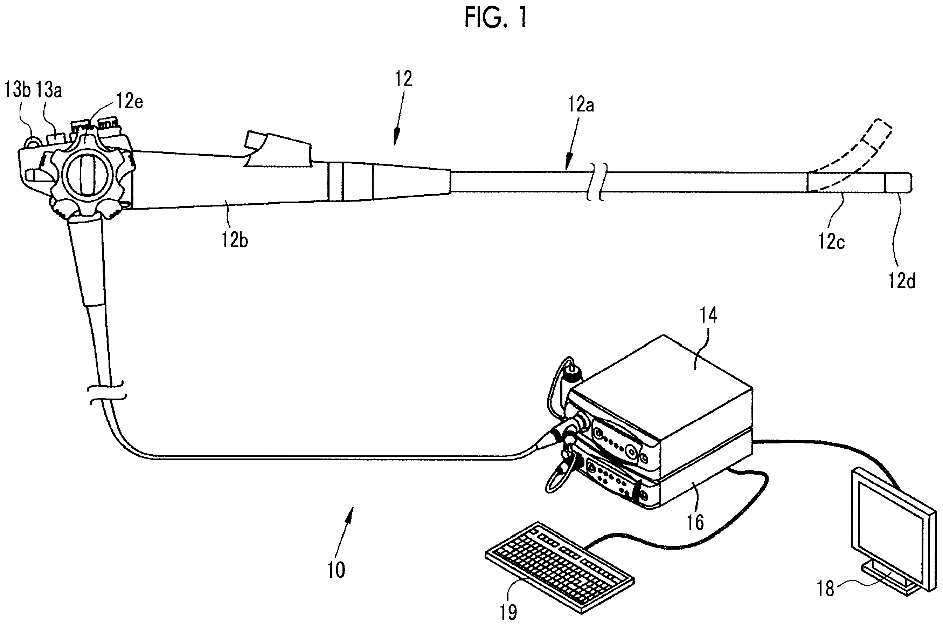

As illustrated in Fig. 1, an endoscope system 10 includes an endoscope 12 which images an observation target, a light source device 14, a processor device 16, a monitor 18 which is a display unit, and a console 19. The endoscope 12 is optically connected to the light source device 14, and is also electrically connected to the processor device 16. The endoscope 12 has an insertion portion 12a inserted into a subject, an operation portion 12b provided at a basal end portion of the insertion portion 12a, a curved portion 12c provided on a distal end side of the insertion portion 12a, and a tip end portion 12d. The curved portion 12c is curved by operating an angle knob 12e of the operation portion 12b. As a result of the curved portion 12c being curved, the tip end portion 12d is directed in a desired direction. The tip end portion 12d is provided with an injection port (not illustrated) for injecting air or water toward an observation target.

The operation portion 12b is provided with a mode changing switch 13a and a zoom operation portion 13b in addition to the angle knob 12e. The mode changing switch 13a is used for an observation mode changing operation. The endoscope system 10 has a normal observation mode and a special observation mode. The normal observation mode is an observation mode in which an observation image (hereinafter, referred to as a normal observation image) with a natural shade is displayed on the monitor 18 by using a captured image obtained by imaging an observation target by using white light as illumination light.

The special observation mode is an observation mode in which an observation image having contrast or a color of a blood vessel or the like which is different from that of a normal observation image is generated and displayed, or an observation mode in which information (hereinafter, referred to as living body information) regarding a living body which is not easily identified at a glance from a normal observation image. The living body information is, for example, numerical information regarding an observation target, such as oxygen saturation or the density of blood vessels.

The special observation mode is an observation mode in which an observation image (hereinafter, referred to as a special observation image) in which contrast, brightness, or a color (hereinafter, referred to as contrast or the like) of a specific tissue or structure is different from that in a normal observation image is generated and displayed. In the special observation mode, one or a plurality of illumination light beams are used in accordance with a specific tissue or structure of which contrast or the like is changed with respect to a normal observation image. Of course, white light may also be used as illumination light in the special observation mode depending on a focused special tissue or structure in diagnosis.

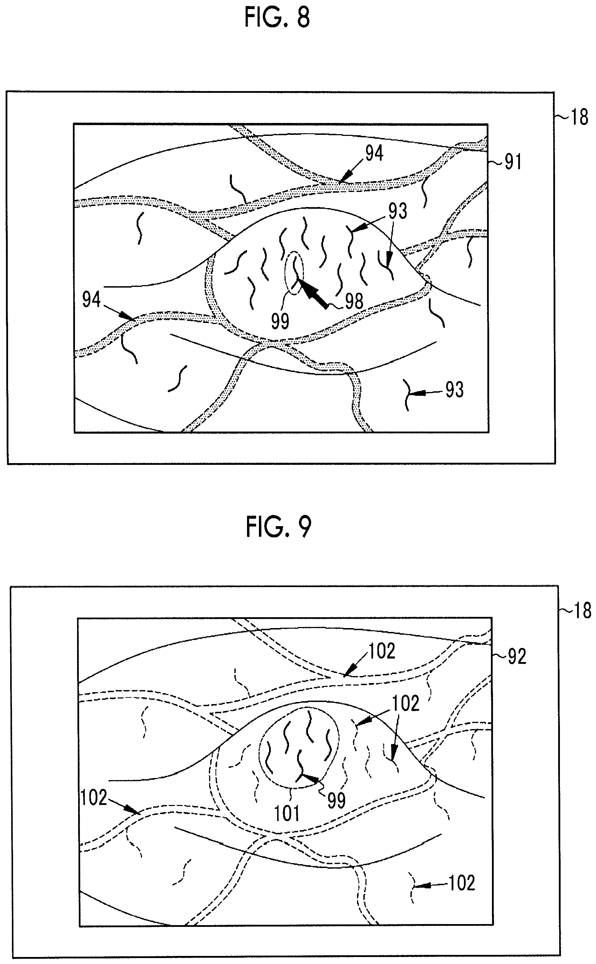

In the special observation mode of the present embodiment, an observation target is imaged by using two types of illumination light having different wavelengths, and thus at least two captured images are acquired. A special observation image (hereinafter, referred to as a blood vessel emphasis image; refer to Figs. 8 and 9) in which a blood vessel at a specific depth is emphasized is generated and displayed by using the two captured images. The emphasis indicates that a blood vessel at a specific depth differs in contrast or the like from blood vessels, mucous membranes, or a structure of a mucosal surface at other depths, or tissues or structures under mucous membranes (hereinafter, referred to as other blood vessels or the like), and a state occurs in which the blood vessel at the specific depth can be differentiated from the other blood vessels or the like. Therefore, the emphasis includes not only a case of directly adjusting contrast or the like of a blood vessel at a specific depth but also a state in which the blood vessel at the specific depth can be relatively differentiated from other blood vessels or the like as a result of suppressing contrast or the like of the other blood vessels or the like.

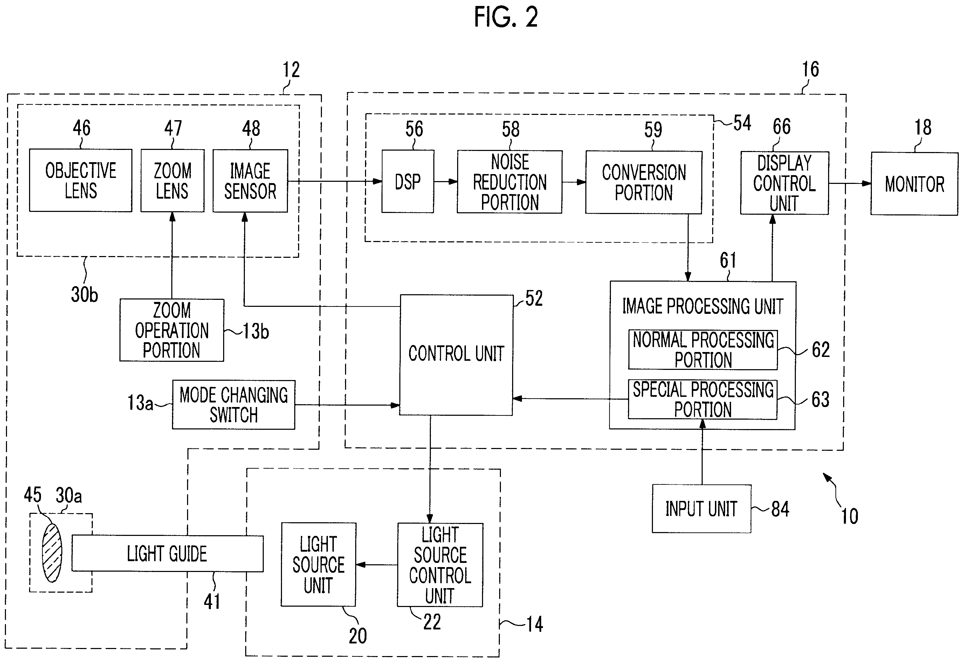

The processor device 16 is electrically connected to the monitor 18 and the console 19. The monitor 18 outputs and displays an observation image in each observation mode and image information or the like attached to the observation target as necessary. The console 19 is one kind of input unit 84 (refer to Fig. 2) which functions as a user interface receiving an input operation such as function setting. The processor device 16 may be connected to an externally attached recording unit (not illustrated) which records an image, image information, or the like.

As illustrated in Fig. 2, the light source device 14 comprises a light source unit 20 which emits illumination light beams having different wavelengths, and a light source control unit 22 which controls driving of the light source unit 20.

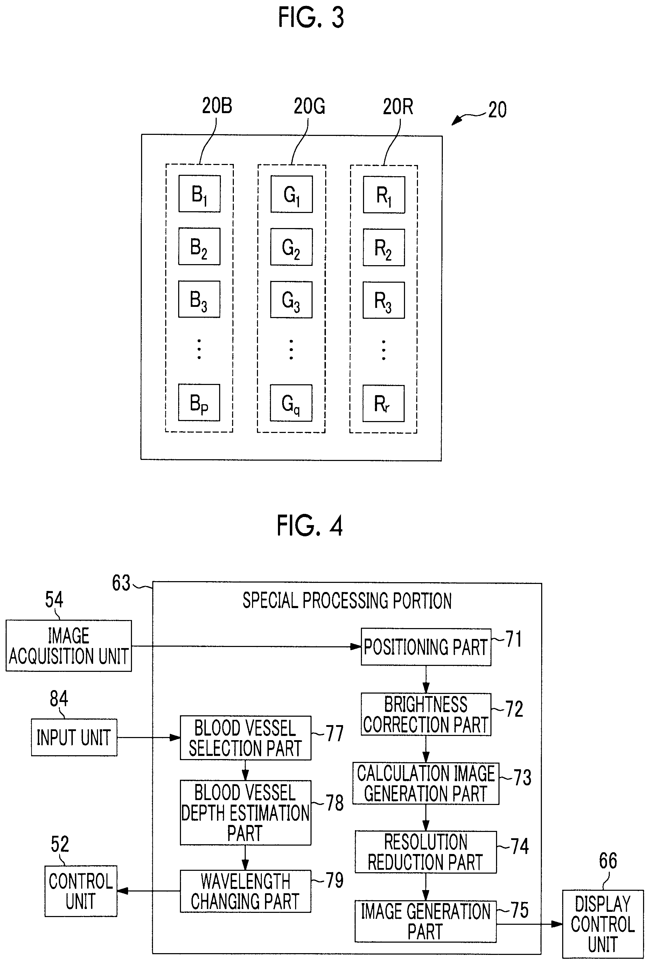

The light source unit 20 includes, for example, one or a plurality of blue light sources emitting blue light, one or a plurality of green light sources emitting green light, and one or a plurality of red light sources emitting red light. In the present embodiment, as illustrated in Fig. 3, the light source unit 20 comprises a blue light source portion 20B having a plurality of blue light sources "B1", "B2", "B3", ..., and "Bp", a green light source portion 20G having a plurality of green light sources "G1", "G2", "G3", ..., and "Gq", and a red light source portion 20R having a plurality of red light sources "R1", "R2", "R3", ..., and "Rr". Each blue light source of the blue light source portion 20B, each green light source of the green light source portion 20G, and each red light source of the red light source portion 20R are, for example, semiconductor light sources such as a light emitting diode (LED), and a light amount and a light emission timing thereof may be separately controlled.

Wavelengths of the respective blue light sources of the blue light source portion 20B are different from each other, and, for example, the wavelengths of the respective blue light sources have a relationship of B1<B2<B3<...<Bp. This is also the same for the respective green light sources of the green light source portion 20G and the respective red light sources of the red light source portion 20R. In other words, in the present embodiment, the wavelengths of the respective green light sources have a relationship of G1<G2<G3<...<Gq, and the wavelength of the respective red light sources have a relationship of R1<R2<R3<...<Rr. A wavelength range of a light source indicates a range from the shortest wavelength of light emitted from the light source to the longest wavelength thereof, and the phrase "different wavelengths" indicates that one or more of a peak wavelength at which a light emission amount is the maximum within a wavelength range, a center wavelength which is the center of a wavelength range, an average wavelength which is an average of the shortest wavelength and the longest wavelength in a wavelength range, the shortest wavelength, or the longest wavelength (hereinafter, referred to as a center wavelength or the like) differ.

A short wavelength (or a long wavelength) indicates that a wavelength is shorter (or longer) than that of a comparison target in a case of being compared in the same reference among the center wavelength or the like. The blue light sources of the blue light source portion 20B may include a violet light source emitting violet light or an ultraviolet light source emitting ultraviolet light, and the red light sources of the red light source portion 20R may include an infrared light source emitting infrared light, as necessary.

The light source unit 20 controls a light emission amount and a light emission timing of each color light source so as to emit a plurality of types of illumination light having different wavelengths as a whole. For example, the light source unit 20 lights one or more of the blue light sources of the blue light source portion 20B, one or more of the green light sources of the green light source portion 20G, and one or more of the red light sources of the red light source portion 20R, so as to emit white light used as illumination light in the normal observation mode. In the special observation mode of the present embodiment, the light source unit 20 selects two light sources from among the respective light sources of the blue light source portion 20B, the green light source portion 20G, and the red light source portion 20R, or selects combinations of the light sources with two different patterns, and causes the light sources to alternately emit light in accordance with an imaging timing (hereinafter, referred to as an imaging frame) of the image sensor 48. In other words, in the special observation mode, the light source unit 20 alternately emits two different types of illumination light of which wavelengths are different from a wavelength of white light, and are different from each other, in accordance with an imaging frame. Illumination light used in the special observation mode is blue light (hereinafter, referred to as B1 light) emitted from the blue light source B1, blue light (hereinafter, referred to as B2 light) emitted from the blue light source B2, and blue light (hereinafter, referred to as B3 light) emitted from the blue light source B3, and two types of light are selected from thereamong and are used according to a depth of a focused blood vessel. Any change of a combination may occur depending on a depth of a focused blood vessel.

The light source unit 20 mixes light beams emitted from the respective light sources with each other by using mirrors or prisms (not illustrated) (including a dichroic mirror or a dichroic prism transmitting or reflecting some components in a wavelength range). The configuration of the light source unit 20 of the present embodiment is only an example, and the light source unit 20 may have any configuration as long as a plurality of kinds of illumination light beams with different wavelengths can be emitted. For example, a lamp such as a xenon lamp, a laser diode (LD), a phosphor, and an optical filter which restricts a wavelength range may be combined with each other as necessary, so as to be used in the light source unit 20. The light source unit 20 is not limited to using the blue light source, the green light source, and the red light source, and may be configured by using a white light source emitting white light, such as a white LED, a light source emitting intermediate light between the blue light source and the green light source, or a light source emitting intermediate light between the green light source and the red light source.

The light source control unit 22 separately controls, for example, a light emission timing and a light emission amount of each light source configuring the light source unit 20 in synchronization with an imaging frame in the image sensor 48 according to an observation mode. The light source unit 20 emits each type of illumination light used in the normal observation mode and the special observation mode under the control of the light source control unit 22.

The illumination light emitted from the light source unit 20 is incident to a light guide 41. The light guide 41 is built into the endoscope 12 and a universal cord, and causes the illumination light to propagate to the tip end portion 12d of the endoscope 12. The universal cord is a cord connecting the endoscope 12, the light source device 14, and the processor device 16 to each other. A multi-mode fiber may be used as the light guide 41. As an example, a fiber cable having small diameters of which a core diameter is 105 µm, a clad diameter is 125 µm, and a diameter including a protection layer serving as a sheath is φ0.3 to 0.5 mm may be used.

The tip end portion 12d of the endoscope 12 is provided with an illumination optical system 30a and an imaging optical system 30b. The illumination optical system 30a has an illumination lens 45, and an observation target is irradiated with illumination light via the illumination lens 45. The imaging optical system 30b has an objective lens 46, a zoom lens 47, and the image sensor 48. The image sensor 48 images an observation target by using reflected light or the like (including, in addition to the reflected light, scattering light, fluorescent light emitted from the observation target, or fluorescent light due to a drug administered to the observation target) of illumination light returning from the observation target via the objective lens 46 and the zoom lens 47. The zoom lens 47 is moved by operating the zoom operation portion 13b, and enlarges or reduces the observation target which imaged by using the image sensor 48 so as to be observed.

The image sensor 48 is a primary color system color sensor, and comprises three types of pixels such as a blue pixel (B pixel) provided with a blue color filter, a green pixel (G pixel) provided with a green color filter, and a red pixel (R pixel) provided with a red color filter. The blue color filter generally transmits therethrough blue light emitted from each blue light source of the blue light source portion 20B. The green color filter generally transmits therethrough green light emitted from each green light source of the green light source portion 20G. The red color filter generally transmits therethrough red light emitted from each red light source of the red light source portion 20R.

In a case where an observation target is imaged by using the image sensor 48, there may be simultaneously obtained three types of captured images such as a blue image (B image) obtained in the B pixel through the imaging, a green image (G image) obtained in the G pixel through the imaging, and a red image (R image) obtained in the R pixel through the imaging. In a case of the normal observation mode, illumination light is white light, and includes blue, green, and red components, and thus a B image, a G image, and an R image may be obtained for each imaging frame.

On the other hand, in the special observation mode of the present embodiment, an obtained captured image differs due to a wavelength of illumination light which is used. For example, in a case where illumination light alternately changes to the B2 light and the B3 light for each imaging frame, substantially, a captured image (hereinafter, referred to as a B2 image) obtained by imaging an observation target in the B pixel by using the B2 light and a captured image (hereinafter, referred to as a B3 image) obtained by imaging the observation target in the B pixel by using the B3 light are alternately obtained. Similarly, in a case where illumination light alternately changes to the B1 light and the B2 light for each imaging frame, substantially, a captured image (hereinafter, referred to as a B1 image) obtained by imaging the observation target in the B pixel by using the B1 light and a B2 image are alternately obtained.

As the image sensor 48, a charge coupled device (CCD) sensor or a complementary metal oxide semiconductor (CMOS) sensor may be used. The image sensor 48 of the present embodiment is a primary color system color sensor, but a complementary color system color sensor may be used. The complementary color system color sensor has, for example, a cyan pixel provided with a cyan color filter, a magenta pixel provided with a magenta color filter, a yellow pixel provided with a yellow color filter, and a green pixel provided with a green color filter. In a case where the complementary color system color sensor is used, images obtained by using the pixels having the respective colors may be converted into the B image, the G image, and the R image through conversion between primary colors and complementary colors. A monochrome sensor in which color filters are not provided may be used as the image sensor 48 instead of a color sensor. In this case, an observation target may be sequentially imaged by using illumination light beams having respective colors such as BGR, and thus the above-described images having the respective colors may be obtained.

The processor device 16 includes a control unit 52, an image acquisition unit 54, an image processing unit 61, and a display control unit 66.

The control unit 52 receives input of a mode changing signal from the mode changing switch 13a, and inputs control signals to the light source control unit 22 and the image sensor 48 so as to change an observation mode. The control unit 52 performs integral control on the endoscope system 10, such as control of synchronization between an illumination light emission timing and an imaging frame.

The image acquisition unit 54 acquires captured images from the image sensor 48. In a case of the normal observation mode, the image acquisition unit 54 acquires a set of the B image, the G image, and the R image for each imaging frame. In a case of the special observation mode, the image acquisition unit 54 acquires a captured image corresponding to illumination light for special observation in each imaging frame for each imaging frame.

The image acquisition unit 54 includes a digital signal processor (DSP) 56, a noise reduction portion 58, and a conversion portion 59, and performs various processes on the acquired images by using the above-described elements.

The DSP 56 performs, on the acquired images, various processes such as a defect correction process, an offset process, a gain correction process, a linear matrix process, a gamma conversion process, a demosaic process, and a YC conversion process, as necessary.

The defect correction process is a process of correcting a pixel value of a pixel corresponding to a defective pixel of the image sensor 48. The offset process is a process of reducing a dark current component from an image subjected to the defect correction process, so as to set an accurate zero level. The gain correction process multiplies the imaged subjected to the offset process by a gain, so as to regulate a signal level of each image. The linear matrix process is a process of increasing color reproducibility of the image subjected to the offset process, and the gamma conversion process is a process of regulating brightness or saturation of the image subjected to the linear matrix process. The demosaic process (also referred to as an equalization process or a synchronization process) is a process of interpolating a pixel value of a missing pixel, and is performed on an image subjected to the gamma conversion process. The missing pixel is a pixel with no pixel value since pixels having other colors are disposed in the image sensor 48 for the purpose of arrangement of color filters. For example, the B image is an image obtained by imaging an observation target in the B pixel, and thus a pixel at a position corresponding to the G pixel or the R pixel of the image sensor 48 does not have a pixel value. The demosaic process is a process of generating pixel values of pixels located at the G pixel and the R pixel of the image sensor 48 interpolating the B image. The YC conversion process is a process of converting the image subjected to the demosaic process into a luminance channel Y, and a color difference channel Cb and a color difference channel Cr.

The noise reduction portion 58 performs a noise reduction process on the luminance channel Y, the color difference channel Cb, and the color difference channel Cr by using, for example, a moving average method or a median filter method. The conversion portion 59 reconverts the luminance channel Y, the color difference channel Cb, and the color difference channel Cr subjected to the noise reduction process into images having the respective colors such as BGR.

The image processing unit 61 includes a normal processing portion 62 and a special processing portion 63. The normal processing portion 62 operates in the normal observation mode, and performs a color conversion process, a color emphasis process, and a structure emphasis process on the B image, the G image, and the R image of one imaging frame having undergone the above-described various processes, so as to generate a normal observation image. The color conversion process is a process of performing a 3×3 matrix process, a grayscale conversion process, and a three-dimensional lookup table (LUT) process on the images having the respective colors such as BGR. The color emphasis process is a process of emphasizing a color of an image, and the structure emphasis process is a process of emphasizing, for example, tissue or a structure of an observation target such as a blood vessel or a pit pattern. The display control unit 66 sequentially acquires normal observation images from the normal processing portion 62, converts the acquired normal observation images to have a form suitable for display, and sequentially outputs and displays the normal observation images to and on the monitor 18. Consequently, in a case of the normal observation mode, a doctor or the like can observe an observation target by using moving normal observation images.

As illustrated in Fig. 4, the special processing portion 63 comprises a positioning part 71, a brightness correction part 72, a calculation image generation part 73, a resolution reduction part 74, an image generation part 75, a blood vessel selection part 77, a blood vessel depth estimation part 78, and a wavelength changing part 79.

The positioning part 71 positions two types of captured images acquired in the special observation mode. For example, in a case where the B2 image and the B3 image are acquired, at least one of the B2 image or the B3 image is moved, rotated, or deformed to be fit to the other image. This is also the same for a case of acquiring the B1 image and the B2 image.

The brightness correction part 72 corrects a brightness of at least one of the two types of captured images such that brightnesses of the two types of captured images positioned by the positioning part 71 have a specific ratio. For example, in a case where the B2 image and the B3 image are acquired, the brightness correction part 72 calculates an average value of pixel values of all pixels of the B2 image and an average value of pixel values of all pixels of the B3 image. The average value of pixel values of all pixels of the B2 image generally indicates the brightness of the mucous membrane of an observation target in the B2 image, and, similarly, the average value of pixel values of all pixels of the B3 image generally indicates the brightness of the mucous membrane of the observation target in the B3 image. In other words, the brightness correction part 72 calculates the brightness of the mucous membrane from each of the B2 image and the B3 image. A gain is applied to the B2 image or the B3 image such that the brightnesses of the mucous membranes are the same as each other, and thus the brightnesses of the B2 image and the B3 image match each other. This is also the same for a case where the B1 image and the B2 image are acquired.

The calculation image generation part 73 performs calculation by using the two types of captured images having undergone positioning and brightness correction, so as to generate a calculation image. In a case where the B2 image and the B3 image are acquired, and the positioning and the brightness correction are performed, the calculation image generation part 73 calculates a difference or a ratio between the B2 image and the B3 image. In the present embodiment, the calculation image generation part 73 performs logarithmic conversion on the B2 image and the B3 image, so as to generate a calculation image Δ in a pixel value of each pixel has a difference value between the B2 image and the B3 image after the logarithmic conversion. This is because, in the B2 image and the B3 image, each pixel has a pixel value proportional to a light reception amount, but has a pixel value proportional to a density through logarithmic conversion, and thus a stable calculation result can be obtained regardless of illuminances of the B2 light and the B3 light. In a case where the B2 image and the B3 image are used without being subjected to logarithmic conversion, a ratio between the B2 image and the B3 image is calculated for each pixel, and thus a calculation image is generated. This is a calculation image generation method useful in a case where it is regarded that there is no difference between illuminances of the B2 light and the B3 light. This is also the same for a case where the B1 image and the B2 image are acquired.

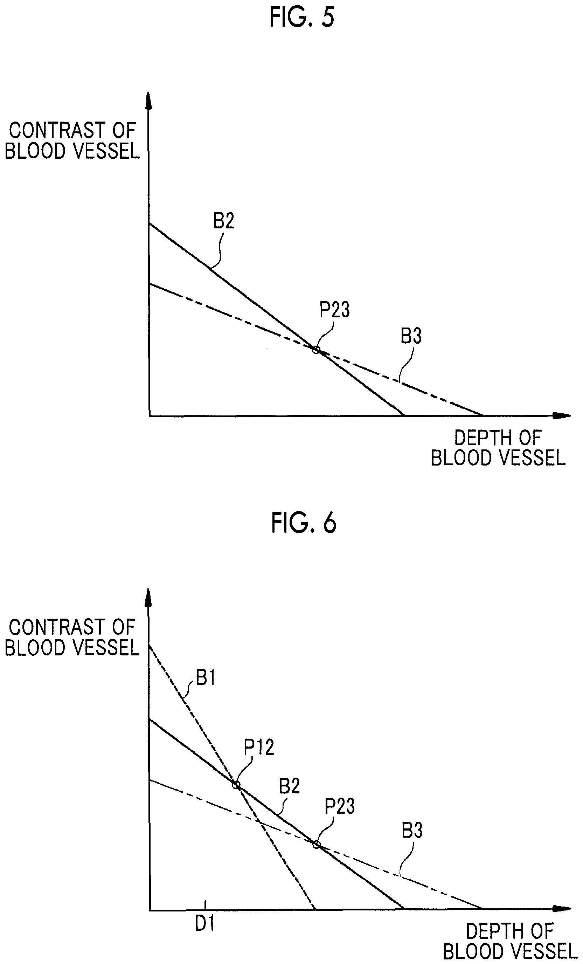

Generating the calculation image Δ corresponds to emphasizing a blood vessel located at a specific depth. For example, as illustrated in Fig. 5, in a case where the B2 light and the B3 light are used illumination light, since the B2 light has a wavelength shorter than that of the B3 light, and thus has a lower dcpth-reaching degree than that of the B3 light, contrast (a ratio of an amount of reflected light from the mucous membrane to an amount of reflected light from a blood vessel) of a blood vessel located at a relatively shallow position is higher than in a case of using the B3 light. Conversely, since the B3 light has a wavelength longer than that of the B2 light, and thus has a higher depth-reaching degree than that of the B2 light, contrast of a blood vessel at a relatively deep position is higher than in a case of using the B2 light. Therefore, in a case where the calculation image Δ is generated by subtracting the B2 image from the B3 image, in the calculation image Δ, a pixel value for a blood vessel at a shallower position than an intersection P23 is great, and a pixel value for a blood vessel at a deeper position than the intersection P23 is small. Conversely, in a case where the calculation image Δ is generated by subtracting the B3 image from the B2 image, in the calculation image Δ, a pixel value for a blood vessel at a shallower position than the intersection P23 is small, and a pixel value for a blood vessel at a deeper position than the intersection P23 is great. Thus, the calculation image Δ generated from the B2 image and the B3 image is an image in which a blood vessel at a shallower position than the intersection P23 or a blood vessel at a deeper position than the intersection P23 is emphasized.

The resolution reduction part 74 is a so-called low-pass filter, and reduces a resolution of the calculation image Δ generated by the calculation image generation part 73. The intensity of resolution reduction of the calculation image Δ in the resolution reduction part 74 is defined by a cutoff frequency. The cutoff frequency is set in advance, and at least an original resolution of the calculation image Δ is reduced.

The image generation part 75 generates an observation image by using either of the two types of captured images acquired by the special processing portion 63, and the calculation image Δ having the reduced resolution. For example, in a case where the special processing portion 63 acquires the B2 image and the B3 image, the image generation part 75 allocates either the B2 image or the B3 image to a luminance channel Y, and allocates the calculation image Δ having the reduced resolution to two color difference channels Cb and Cr, so as to generate an observation image. As is clear from the fact that the calculation image Δ corresponds to emphasizing a blood vessel located at a specific depth, as described above, the observation image generated by the image generation part 75 is a blood vessel emphasis image in which the blood vessel at the specific depth is emphasized. For example, in a case where the B2 light and the B3 light are used as illumination light, in a blood vessel emphasis image 91 (refer to Fig. 8) generated by the image generation part 75, a blood vessel at a shallower position than the intersection P23 and a blood vessel at a deeper position than the intersection P23 are displayed in different colors.

A captured image to be allocated to the luminance channel Y differs depending on a depth of an emphasized blood vessel. For example, in a case where a blood vessel at a shallower position than the intersection P23 is emphasized, the calculation image generation part 73 generates the calculation image Δ by subtracting the B2 image from the B3 image, and the image generation part 75 allocates the B2 image to the luminance channel Y. Conversely, in a case where a blood vessel at a deeper position than the intersection P23 is emphasized, the calculation image generation part 73 generates the calculation image Δ by subtracting the B3 image from the B2 image, and the image generation part 75 allocates the B3 image to the luminance channel Y. In other words, the image generation part 75 allocates a captured image in which contrast of a blood vessel to be emphasized is higher to the luminance channel Y. The image generation part 75 may multiply the color difference channels Cb and Cr by a coefficient α and a coefficient β (where α≠β), respectively, in order to allocate the calculation image Δ to the color difference channels Cb and Cr. This is because a tint is matched with that of a blood vessel emphasis image which is generated and displayed by an endoscope system of the related art.

The image generation part 75 inputs the generated blood vessel emphasis image to the display control unit 66. In a case of the special observation mode, the display control unit 66 sequentially acquires blood vessel emphasis images from the image generation part 75, converts the acquired blood vessel emphasis images to have a form suitable for display, and sequentially outputs and displays the blood vessel emphasis images to and on the monitor 18. Consequently, in a case of the special observation mode, a doctor or the like can observe an observation target by using moving blood vessel emphasis images.

The blood vessel selection part 77 selects a focused blood vessel which is focused in diagnosis, from a captured image of an observation target imaged by using illumination light in the special observation mode, or from an observation image generated by using a captured image of an observation target imaged by using illumination light in the special observation mode. For example, the blood vessel selection part 77 selects a focused blood vessel from the B2 image or the B3 image of an observation target imaged by using the B2 light or the B3 light in the special observation mode, or from a blood vessel emphasis image generated by using the B2 image or the B3 image.

In the present embodiment, a doctor or the like views the blood vessel emphasis image displayed on the monitor 18, and inputs an instruction for designating a focused blood vessel to be selected to the blood vessel selection part 77 by using an input unit 84, so as to designate the focused blood vessel. Thus, the blood vessel selection part 77 selects the focused blood vessel from the blood vessel emphasis image displayed on the monitor 18 or from the captured image used to generate the blood vessel emphasis image displayed on the monitor 18, on the basis of the instruction from the input unit 84. The input unit 84 is, for example, an input operation screen (graphical user interface (GUI)) displayed on the monitor 18, and is operated by using the console 19 or the like.

The blood vessel depth estimation part 78 estimates a depth of the focused blood vessel selected by the blood vessel selection part 77. More specifically, the blood vessel depth estimation part 78 estimates a depth of the focused blood vessel by using contrast, brightness, or a color of the focused blood vessel selected by the blood vessel selection part 77. A depth and contrast of a blood vessel have a substantially constant relationship according to a wavelength of illumination light which is used (refer to Fig. 5), and thus a depth of the focused blood vessel may be estimated on the basis of contrast or brightness of the focused blood vessel in a captured image. Similarly, in a blood vessel emphasis image, a depth of the selected focused blood vessel may be estimated on the basis of contrast, brightness, or a color thereof.

The wavelength changing part 79 changes a wavelength of illumination light by using the depth of the focused blood vessel estimated by the blood vessel depth estimation part 78. Specifically, in a case where a wavelength of illumination light which is used is determined by using the depth of the focused blood vessel, the wavelength changing part 79 inputs a control signal for designating a wavelength of illumination light which is used or a light source which is used to the light source control unit 22 via the control unit 52, so as to change a wavelength of the illumination light. "Changing a wavelength of illumination light" indicates that illumination light which is used is changed to illumination light of which one or more of the center wavelength or the like (a peak wavelength, a center wavelength, an average wavelength, the shortest wavelength, or the longest wavelength) differ. Particularly, in a case where a plurality of types of illumination light are used, "changing a wavelength of illumination light" indicates that wavelengths of one or more of the plurality of types of illumination light are changed.

The wavelength changing part 79 changes a wavelength of illumination light to a shorter wavelength as the depth of the focused blood vessel estimated by the blood vessel depth estimation part 78 becomes smaller. "Changing a wavelength of illumination light to a short wavelength" indicates that illumination light which is used is changed to illumination light of which one or more of the center wavelength or the like are short. In a case where a plurality of types of illumination light are used, "changing a wavelength of illumination light to a short wavelength" indicates that wavelengths of one or more of the plurality of types of illumination light are changed to short wavelengths, and an average value, a median value, the maximum value, or the minimum value (hereinafter, referred to as an average value or the like) of the center wavelengths or the like of a plurality of types of changed illumination light is smaller than the average value or the like of center wavelengths of the plurality of types of original illumination light.

For example, as illustrated in Fig. 6, in a case where the B2 light and the B3 light are used as illumination light, and a blood vessel at a depth "D1" smaller than the depth of the intersection P23 is selected as a focused blood vessel, the wavelength changing part 79 changes illumination light from a combination of the B2 light and the B3 light to a combination of the B1 light and the B2 light. This is because, in a case where a focused blood vessel is located at a shallow position, a wavelength of illumination light is changed to a short wavelength, and thus the focused blood vessel and a blood vessel which is located near the focused blood vessel and is located at a deeper position than the focused blood vessel can be clearly differentiated from each other so as to be emphasized. For example, in a case where an estimated depth of the focused blood vessel is the depth "D1" smaller than the depth of the intersection P23, and a combination of the B2 light and the B3 light is continuously used as illumination light, the focused blood vessel located near the depth D1 and a blood vessel within a range of a depth from an intersection P12 to the intersection P23 have an identical color in a blood vessel emphasis image. However, in a case where illumination light which is used is changed from a combination of the B2 light and the B3 light to a combination of the B1 light and the B2 light (that is, changed to a short wavelength), the focused blood vessel located near the depth D1 and the blood vessel within a range of a depth from the intersection P12 to the intersection P23 have different colors in the blood vessel emphasis image, and thus the focused blood vessel located near the depth D1 can be more clearly emphasized.

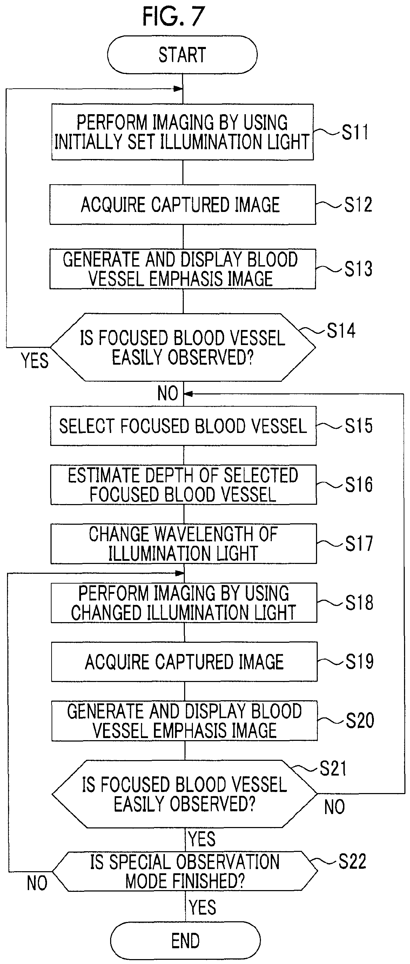

Next, a description will be made of a flow of a series of operations in the special observation mode of the present embodiment with reference to a flowchart in Fig. 7. First, in a case where an observation mode is changed to the special observation mode by operating the mode changing switch 13a, the endoscope system 10 images an observation target by using initially set illumination light (S11), and acquires a captured image (S12). In the present embodiment, the B2 light and the B3 light are initially set illumination light, and, in a case where an observation mode is changed to the special observation mode, the observation target is imaged by alternately using the B2 light and the B3 light for each imaging frame, and the image acquisition unit 54 acquires the B2 image and the B3 image.

In a case where the image acquisition unit 54 acquires the B2 image and the B3 image, the positioning part 71 positions the B2 image and the B3 image, and the brightness correction part 72 corrects brightnesses of the B2 image and the B3 image after being positioned. The calculation image generation part 73 generates the calculation image Δ by using the B2 image and the B3 image having undergone the brightness correction, and the resolution reduction part 74 reduces a resolution of the calculation image Δ. Thereafter, the image generation part 75 allocates the B2 image or the B3 image to the luminance channel Y, and allocates the calculation image Δ having the reduced resolution to the two color difference channels Cb and Cr, so as to generate the blood vessel emphasis image 91 (refer to Fig. 8), and displays the generated blood vessel emphasis image 91 on the monitor 18 via the display control unit 66 (S13). As illustrated in Fig. 8, in the blood vessel emphasis image 91 obtained by using the B2 light and the B3 light as illumination light, a blood vessel 93 shallower than the intersection P23 (refer to Fig. 5 or 6) and a blood vessel 94 deeper than the intersection P23 have different colors so as to be emphasized.

In a case where the blood vessel emphasis image 91 is displayed on the monitor 18 in the above-described way, a doctor or the like views the blood vessel emphasis image 91, and checks whether or not the focused blood vessel which is focused in diagnosis is easily observed (S14). In a case where the focused blood vessel is already easily observed in the blood vessel emphasis image 91 obtained by using the B2 light and the B3 light as illumination light, the doctor or the like is not required to reselect the focused blood vessel by using the input unit 84, and thus the endoscope system 10 continuously update the blood vessel emphasis image 91 by using the B2 light and the B3 light as illumination light (S14: YES).

On the other hand, in a case where it is determined that the focused blood vessel is hardly observed while viewing the blood vessel emphasis image 91 obtained by using the B2 light and the B3 light as illumination light, or in a case where the focused blood vessel is desired to be more clearly emphasized and observed than other blood vessels or the like (S14: NO), the doctor or the like clicks the focused blood vessel by using a blood vessel selection pointer 98 which is one of GUIs from the console 19. In the present embodiment, it is assumed that the doctor or the like clicks a single blood vessel 99 surrounded by a dashed line from among the blood vessels 93 shallower than the intersection P23, as a focused blood vessel.

As described above, in a case where the doctor or the like clicks the blood vessel 99, the console 19 corresponding to the input unit 84 inputs a signal indicating a position of the blood vessel 99 in the blood vessel emphasis image 91 to the blood vessel selection part 77 as an instruction for designating the focused blood vessel. Thus, the blood vessel selection part 77 selects the blood vessel 99 as the focused blood vessel from the blood vessel emphasis image 91 (S15).

In a case where the blood vessel selection part 77 selects the blood vessel 99 as the focused blood vessel, the blood vessel depth estimation part 78 estimates a depth of the blood vessel 99 on the basis of contrast or the like of the blood vessel 99 selected by the blood vessel selection part 77 (S16). The wavelength changing part 79 changes a wavelength of the illumination light by using the depth of the blood vessel 99 estimated by the blood vessel depth estimation part 78 (S17). In the present embodiment, it is assumed that the depth of the blood vessel 99 selected as the focused blood vessel is a blood vessel shallower than the intersection P23. Thus, the wavelength changing part 79 changes the illumination light from a combination of the B2 light and the B3 light to a combination of the B1 light and the B2 light having shorter wavelengths.

In a case where the wavelength changing part 79 changes a wavelength of the illumination light, the endoscope system 10 images the observation target by using the changed illumination light (S18), and the image acquisition unit 54 acquires a new captured image (S19). In the present embodiment, the observation target is imaged by using the B1 light and the B2 light, and the image acquisition unit 54 acquires the B1 image and the B2 image.

In a case where the image acquisition unit 54 acquires the B1 image and the B2 image, the positioning part 71 positions the B1 image and the B2 image, and the brightness correction part 72 corrects brightnesses of the B1 image and the B2 image after being positioned. The calculation image generation part 73 generates the calculation image Δ by using the B1 image and the B2 image having undergone the brightness correction, and the resolution reduction part 74 reduces a resolution of the calculation image Δ. Thereafter, the image generation part 75 allocates the B1 image or the B2 image to the luminance channel Y, and allocates the calculation image Δ having the reduced resolution to the two color difference channels Cb and Cr, so as to generate a blood vessel emphasis image 92 (refer to Fig. 9), and displays the generated blood vessel emphasis image 92 on the monitor 18 via the display control unit 66 (S20). As illustrated in Fig. 9, in the blood vessel emphasis image 92 obtained by changing the illumination light to the B1 light and the B2 light, blood vessels 101 (including the blood vessel 99 which is the focused blood vessel) shallower than the intersection P12 (refer to Fig. 6) and blood vessels 102 deeper than the intersection P12 have different colors so as to be emphasized.

In a case where the blood vessel emphasis image 91 (refer to Fig. 8) obtained by using the B2 light and the B3 light as illumination light is compared with the blood vessel emphasis image 92 (refer to Fig. 9) obtained by using the B1 light and the B2 light as illumination light, the blood vessels are differentiated from each other by colors in the vicinity of the depth of the blood vessel 99 which is a focused blood vessel in the blood vessel emphasis image 92 obtained by using the B1 light and the B2 light as illumination light, and thus the focused blood vessel can be more appropriately emphasized than in the blood vessel emphasis image 91 obtained by using the B2 light and the B3 light as illumination light.

In the blood vessel emphasis image 92 generated and displayed after the illumination light is changed (S17), in a case where the doctor determines that the focused blood vessel is not appropriately emphasized, or desires the focused blood vessel to be more clearly emphasized than other blood vessels (S21: NO), a focused blood vessel may be selected as appropriate as described above (S15), a wavelength of the illumination light may be changed (S17), and a more appropriate blood vessel emphasis image may be generated and displayed (S20). In a case where the focused blood vessel is appropriately emphasized (S21: YES), the endoscope system 10 repeatedly images the observation target by using illumination light of which a wavelength is changed, so as to generate and display the blood vessel emphasis image 92 until the special observation mode is finished (S22).

As described above, the endoscope system 10 changes a wavelength of illumination light by using a depth of a focused blood vessel (the blood vessel 99), and can thus emphasize the focused blood vessel to be differentiated from other blood vessels or the like. Thus, the focused blood vessel can be more easily observed than in the endoscope system 10 of the related art.

As in the first embodiment, in the endoscope system 10, the image acquisition unit 54 may acquire a plurality of observation target images (captured images) obtained by imaging the observation target at different timings by using illumination light beams having different wavelengths. In the first embodiment, the blood vessel 99 which is a focused blood vessel is selected by using the blood vessel emphasis image 91 generated by the image generation part 75, but the blood vessel selection part 77 may select a blood vessel by using a single or plural images among a plurality of observation target images (captured images) obtained by imaging the observation target at different timings by using illumination light beams having different wavelengths. The blood vessel depth estimation part 78 may estimate a depth of a focused blood vessel selected by the blood vessel selection part 77 by using one or more of a plurality of observation target images (captured images) obtained by imaging the observation target at different timings by using illumination light beams having different wavelengths.

In a case where the endoscope system 10 has a plurality of observation modes in which the type of illumination light which is used or a combination of illumination light beams differs, the blood vessel selection part 77 preferably selects a predefined blood vessel in each observation mode. For example, in the first embodiment, illumination light having any wavelength may be used in the special observation mode, but a wavelength of illumination light which is used may be restricted. For example, an observation mode may be provided in which a specific tissue such as a so-called superficial blood vessel is emphasized by using blue light and green light. In this case, instead of changing a wavelength of illumination light to any wavelength as in the first embodiment, the wavelength of illumination light is preferably changed within a range of "blue light" and a range of "green light" of which the use is defined. As mentioned above, in a case where a wavelength of illumination light is changed within the range of illumination light of which the use is defined, specific tissue to be emphasized can be reliably emphasized, and the specific tissue can be emphasized with high accuracy as in the first embodiment.

### [Second Embodiment]

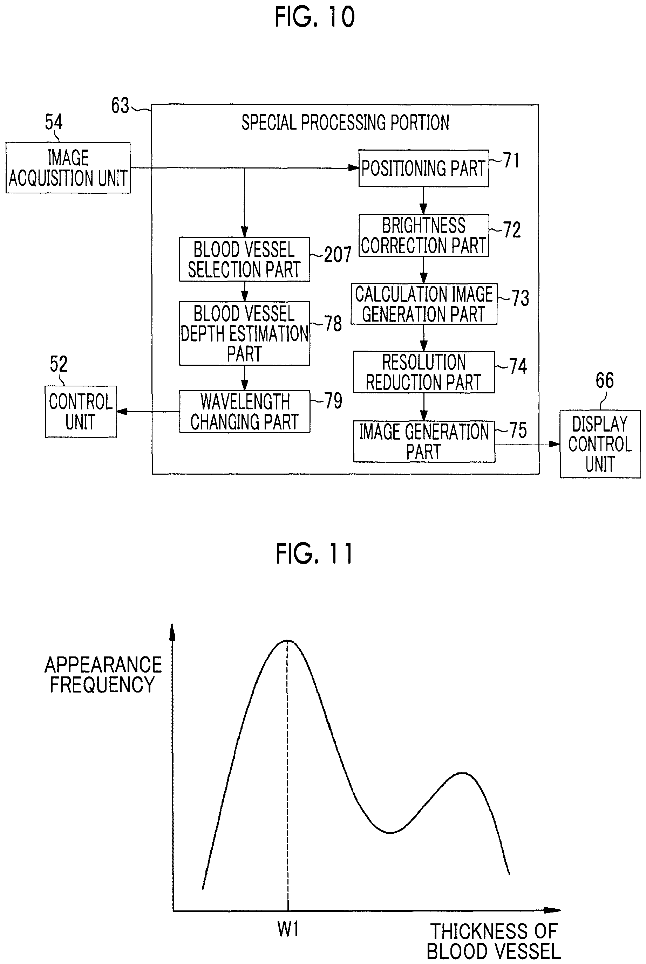

In the first embodiment, the blood vessel selection part 77 receives an instruction for designating a focused blood vessel from the input unit 84, and thus selects the focused blood vessel, but the focused blood vessel can be automatically selected. As illustrated in Fig. 10, in a case where a blood vessel selection part 207 automatically selects a focused blood vessel without receiving input from the input unit 84, the input unit 84 is not necessary. The blood vessel selection part 207 may classify blood vessels which are selectable on the basis of thicknesses thereof as illustrated in Fig. 11, for example, from a captured image (an image of an observation target) or from an observation image generated by using the captured image, and may select a blood vessel having a thickness "W1" of which an appearance frequency is highest, as a focused blood vessel.

In a case where observation is performed by using the endoscope system 10, generally, the observation is performed by adjusting an observation distance or the like such that a focused blood vessel desired to be observed is frequently observed in order to perform diagnosis. A thickness of a blood vessel and a depth of the blood vessel have a correlation therebetween. Thus, in a case where a blood vessel at a specific depth is a focused blood vessel, as described above, and the blood vessel selection part 207 classifies blood vessels on the basis of thicknesses thereof, and selects a blood vessel having a thickness "W1" of which an appearance frequency is highest, as a focused blood vessel, it is possible to substantially automatically and accurately select the focused blood vessel.

### [Third Embodiment]

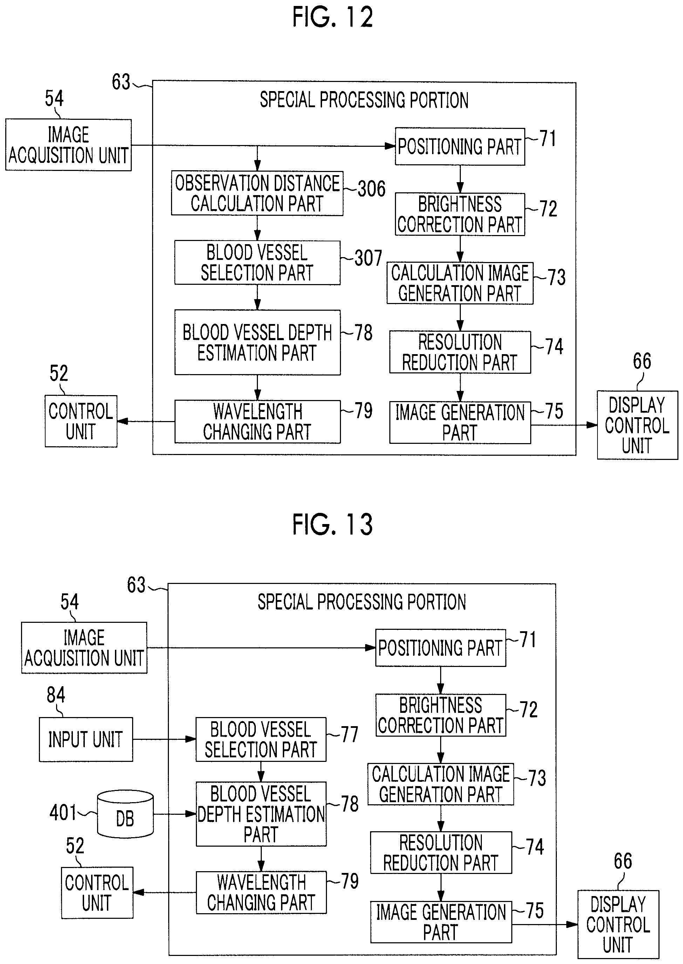

In the second embodiment, the blood vessel selection part 207 classifies blood vessels on the basis of thicknesses thereof, and automatically selects a blood vessel having a thickness "W1" of which an appearance frequency is highest, as a focused blood vessel, but a focused blood vessel may be automatically selected by using an observation distance. In this case, as illustrated in Fig. 12, the special processing portion 63 includes an observation distance calculation part 306 which calculates an observation distance indicating a distance at which an observation target is imaged from a captured image (an image of the observation target) or an observation image generated by using the captured image, and a blood vessel selection part 307 which determines a blood vessel to be selected as a focused blood vessel by using the observation distance calculated by the observation distance calculation part 306. The observation distance calculation part 306 calculates an observation distance on the basis of, for example, the brightness of the mucous membrane in a captured image or an observation image, or an operation state of the zoom operation portion 13b (to what extent zooming is applied).

In a case where a focused blood vessel is automatically selected by using an observation distance as mentioned above, preferably, the blood vessel selection part 307 classifies blood vessels on the basis of depths thereof by using a captured image or an observation image, and selects, as a focused blood vessel, a blood vessel of which a depth becomes smaller as an observation distance becomes shorter. This corresponds to a state in which a narrow region is enlarged and observed through enlargement of the observation distance as an observation distance becomes shorter, and this is because a doctor or the like tries to clearly observe a blood vessel at a shallow position near a mucosal surface. Classification of blood vessels in a captured image or an observation image on the basis of thicknesses thereof, performed by the blood vessel selection part 307, is relative classification in a single captured image or a single observation image. Thus, the blood vessel depth estimation part 78 estimates a depth of a blood vessel selected as a focused blood vessel by the blood vessel selection part 307 in the same manner as in the first embodiment.

In the third embodiment, the blood vessel selection part 307 selects, as a focused blood vessel, a blood vessel of which a depth becomes smaller as an observation distance becomes shorter, but the blood vessel selection part 307 may classify blood vessels on the basis of thicknesses thereof by using a captured image or an observation image, and may select a blood vessel of which a thickness becomes smaller as a focused blood vessel as an observation distance becomes shorter. As mentioned above, the reason of selecting a blood vessel of which a thickness becomes smaller as a focused blood vessel as an observation distance becomes shorter is that this corresponds to a state in which a narrow region is enlarged and observed through enlargement of the observation distance as an observation distance becomes shorter, and this is because a doctor or the like tries to clearly observe a relatively thin blood vessel near a mucosal surface. The blood vessel selection part 307 may classify blood vessels on the basis of depths and thicknesses thereof by using a captured image or an observation image, and may select a blood vessel of which a depth and a thickness become smaller as a focused blood vessel as an observation distance becomes shorter.

In the first to third embodiments, the blood vessel depth estimation part 78 estimates a depth of a focused blood vessel by using contrast, brightness, or a color of the focused blood vessel selected by the blood vessel selection part 77 or the like, but, as illustrated in Fig. 13, the blood vessel depth estimation part 78 may estimate a depth of a focused blood vessel by using a database 401. The database 401 may accumulate information in which a wavelength of illumination light is correlated with contrast, brightness, or a color thereof, and may accumulate a captured image or an observation image obtained by using illumination light having each wavelength. In a case where the blood vessel depth estimation part 78 estimates a depth of a focused blood vessel by using the database 401, the depth of the focused blood vessel is estimated by comparing contrast, brightness, or a color of the focused blood vessel selected by the blood vessel selection part 77, or a captured image or an observation image with information accumulated in the database 401.

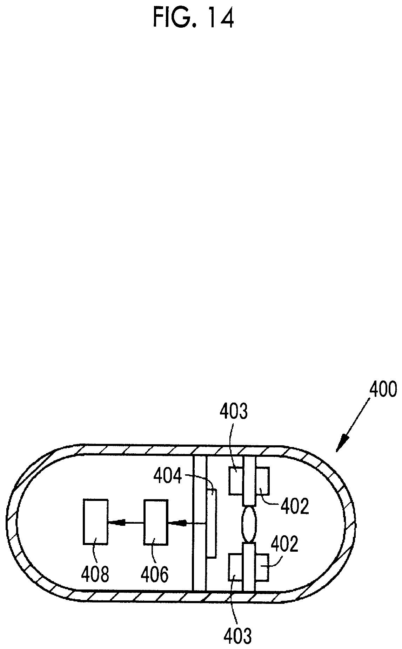

In the first to third embodiments, the present invention is implemented in the endoscope system in which the endoscope 12 provided with the image sensor 48 is inserted into a subject and observation is performed, but the present invention is suitable for a capsule endoscope system. As illustrated in Fig. 14, for example, a capsule endoscope system includes at least a capsule endoscope 700 and a processor device (not illustrated).

The capsule endoscope 700 includes a light source unit 702, a control unit 703, an image sensor 704, an image processing unit 706, and a transmission/reception antenna 708. The light source unit 702 corresponds to the light source unit 20. The control unit 703 functions in the same manner as the light source control unit 22 and the control unit 52. The control unit 703 performs communication with the processor device of the capsule endoscope system in a wireless manner by using the transmission/reception antenna 708. The processor device of the capsule endoscope system is substantially the same as the processor device 16 of the first to third embodiments, but the image processing unit 706 corresponding to the image acquisition unit 54 and the image processing unit 61 is provided in the capsule endoscope 700, and generated observation images such as the blood vessel emphasis images 91 and 92 are transmitted to the processor device via the transmission/reception antenna 708. The image sensor 704 is configuration in the same manner as the image sensor 48.

### Explanation of References

10, 201, and 301: endoscope system
12: endoscope
12a: insertion portion
12b: operation portion
12c: curved portion
12d: tip end portion
12e: angle knob
13a: switch
13b: zoom operation portion
14: light source device
16: processor device
18: monitor
19: console
20 and 702: light source unit
20B: blue light source portion
20G: green light source portion
20R: red light source portion
22: light source control unit
30a: illumination optical system
30b: imaging optical system
41: light guide
45: illumination lens
46: objective lens
47: zoom lens
48 and 704: image sensor
52 and 703: control unit
54: image acquisition unit
56: DSP
58: noise reduction portion
59: conversion portion
61 and 706: image processing unit
62: normal processing portion
63: special processing portion
66: display control unit
71: positioning part
72: brightness correction part
73: calculation image generation part
74: resolution reduction part
75: image generation part
77, 207, and 307: blood vessel selection part
78: estimation part
79: wavelength changing part
84: input unit
91 and 92: blood vessel emphasis image
93, 94, 99, 101, and 102: blood vessel
98: blood vessel selection pointer
306: observation distance calculation part
401: database
700: capsule endoscope
708: transmission/reception antenna
B1, B2, B3,..., and Bp: blue light source
Cb and Cr: color difference channel
P12 and P23: intersection
Y: luminance channel

## Claims

1. An endoscope system comprising:
a light source unit that is able to emit a plurality of types of illumination light having different wavelengths;
a blood vessel selection part that selects a blood vessel from an image of an observation target imaged by using the illumination light, or from an image generated on the basis of an image of an observation target imaged by using the illumination light;
a blood vessel depth estimation part that estimates a depth of the blood vessel selected by the blood vessel selection part; and
a wavelength changing part that changes a wavelength of the illumination light by using the depth of the blood vessel estimated by the blood vessel depth estimation part.

2. The endoscope system according to claim 1,
wherein the wavelength changing part changes the wavelength of the illumination light to a shorter wavelength as the estimated depth of the blood vessel becomes smaller.

3. The endoscope system according to claim 1 or 2,
wherein the blood vessel selection part classifies blood vessels which are selectable from an image of the observation target or from an image generated on the basis of an image of the observation target imaged by using the illumination light, on the basis of thicknesses of the blood vessels, and selects a blood vessel having a thickness of which an appearance frequency is highest.

4. The endoscope system according to claim 1 or 2, further comprising:
an observation distance calculation part that calculates an observation distance indicating a distance at which the observation target is imaged from an image of the observation target or from an image generated on the basis of an image of the observation target imaged by using the illumination light,
wherein the blood vessel selection part determines a blood vessel to be selected by using the observation distance.

5. The endoscope system according to claim 4,
wherein the blood vessel selection part selects a blood vessel at a shallower position as the observation distance becomes shorter.

6. The endoscope system according to claim 4 or 5,
wherein the blood vessel selection part selects a thinner blood vessel as the observation distance becomes shorter.

7. The endoscope system according to claim 1 or 2, further comprising:
an input unit that inputs an instruction for designating a blood vessel to be selected to the blood vessel selection part.

8. The endoscope system according to claim 7,
wherein the input unit is a graphical user interface.

9. The endoscope system according to claim 1 or 2,
wherein the endoscope system has a plurality of observation modes in which the type of illumination light which is used or a combination of illumination light beams differs, and
wherein the blood vessel selection part selects a predefined blood vessel in each of the observation modes.

10. The endoscope system according to any one of claims 1 to 9,
wherein the blood vessel depth estimation part estimates a depth of the blood vessel selected by the blood vessel selection part by using a database.

11. The endoscope system according to any one of claims 1 to 10,
wherein the blood vessel depth estimation part estimates a depth of the blood vessel selected by the blood vessel selection part by using contrast, brightness, or a color of the blood vessel selected by the blood vessel selection part.

12. The endoscope system according to any one of claims 1 to 11, further comprising:
an image acquisition unit that acquires a plurality of images of the observation target obtained by imaging the observation target at timings at which the plurality of types of the illumination light having different wavelengths are applied by using the plurality of illumination light having different wavelengths and applied at different timings,
wherein the blood vessel selection part selects a blood vessel by using a single or plural images among the plurality of images of the observation target, and the blood vessel depth estimation part estimates a depth of the blood vessel selected by the blood vessel selection part by using a single or plural images among the plurality of images of the observation target.

13. A processor device comprising:
a blood vessel selection part that selects a blood vessel from an image of an observation target imaged by using illumination light, or from an image generated on the basis of an image of an observation target imaged by using the illumination light;
a blood vessel depth estimation part that estimates a depth of the blood vessel selected by the blood vessel selection part; and
a wavelength changing part that changes a wavelength of the illumination light by using the depth of the blood vessel estimated by the blood vessel depth estimation part.

14. An endoscope system operation method for an endoscope system including a light source unit that is able to emit a plurality of types of illumination light having different wavelengths, the endoscope system operation method comprising:
a step of causing a blood vessel selection part to select a blood vessel from an image of an observation target imaged by using the illumination light, or from an image generated on the basis of an image of an observation target imaged by using the illumination light;
a step of causing a blood vessel depth estimation part to estimate a depth of the blood vessel selected by the blood vessel selection part; and
a step of causing a wavelength changing part to change a wavelength of the illumination light by using the depth of the blood vessel estimated by the blood vessel depth estimation part.
